# EUROPEAN PATENT APPLICATION

(11) **EP 4 722 391 A1**
(43) Date of publication of application: **08.04.2026**
(21) Application number: 24815295.1
(22) Date of filing: 21.05.2024
(51) Int. Cl.: C12Q 1/689, C12Q 1/6844, C12N 15/31

(54) **PRIMER SET, DETECTION METHOD AND DETECTION KIT FOR DETECTION OF CARBAPENEMASE-PRODUCING ENTEROBACTERIACEAE BACTERIUM**

(30) Priority: 01.06.2023 JP 2023091020
(71) Applicant: Keio University, Tokyo, 108-8345 (JP)
(72) Inventor: MIKITA, Kei, Tokyo 160-8582 (JP)
(74) Representative: Dolphin, Kirsty Mairi
(86) International application number: PCT/JP2024/018641
(87) International publication number: WO 2024/247811

(57) **Abstract**

A primer set for detecting carbapenemase-producing Enterobacteriaceae is provided. The primer set includes one or more LAMP primer sets or one or more LAMP DNA chromatography primer sets that are respectively specific to various bacterial types, blaNDM-type, blaOXA-type, blaIMP-type, blaKPC-type, and blaVIM-type, of carbapenemase genes of carbapenemase-producing Enterobacteriaceae.

## Description

### TECHNICAL FIELD

The present invention relates to a primer set for detecting carbapenemase-producing Enterobacteriaceae, a detection method, and a detection kit.

### BACKGROUND OF THE INVENTION

Carbapenemase-producing Enterobacteriaceae (CPE) are bacteria of the family Enterobacteriaceae that are resistant to carbapenem antibiotics, and outbreak of CPE is a worldwide problem.

There are many bacterial types of CPE, such as NDM, OXA, IMP, KPC, VIM, and the like. Among IMP CPE, IMP-6 or the like includes strains called "stealth type", which do not necessarily exhibit resistance in a result of drug susceptibility testing for carbapenems, and such strains may be missed in screening performed using only carbapenems as an index (see, for example, Non-Patent Document 1).

Although the number of cases of CPE detected in Japan is still low, cases where CPE is detected from a patient who has a history of hospitalization in endemic areas such as overseas, and cases of nosocomial infections have been reported. In Japan, CPE was designated as Category V Infectious Disease in 2014. As a method of promptly and assuredly detecting CPE, a gene amplification testing method, such as PCR, has been desired.

CPE have characteristics such that CPE stay in intestinal microbiota over a long period, and lead to poor outcomes of a treatment. In addition, CPE have resistance genes having high resistance to carbapenem antibiotics on transmissible plasmids, and the resistant genes are transferred to the same or different species of Enterobacteriaceae bacteria, as the plasmids are transmitted. Thus, early detection of CPE is important for clinical pathology and nosocomial infection countermeasures.

### RELATED-ART DOCUMENT

### Non-Patent Documents

Non-Patent Document 1: Infectious Agents Surveillance Report (IASR) Vol. 40 p 22-24: 2019

### SUMMARY OF THE INVENTION

### PROBLEMS TO BE SOLVED BY THE INVENTION

The present invention aims to solve the above various problems existing in the related art, and to achieve the following object. Specifically, the present invention has an object to provide a primer set for detecting carbapenemase-producing Enterobacteriaceae, a detection method, and a detection kit, which can specifically detect carbapenemase-producing Enterobacteriaceae even in a system in which a plurality of microbial species are present.

### MEANS FOR SOLVING THE PROBLEMS

The means for solving the above problems are as follows.
<1> A primer set for detecting carbapenemase-producing Enterobacteriaceae, the primer set including:
   at least one selected from the group consisting of
      (1) a first primer set specific to an NDM-type carbapenemase gene, blaNDM, the first primer set including four oligonucleotides having having base sequences of SEQ ID NOs: 1 to 4, respectively,
      (2) a second primer set specific to an OXA-type carbapenemase gene, blaOXA, the second primer set including six oligonucleotides having base sequences of SEQ ID NOs: 5 to 10, respectively,
      (3) a third primer set specific to an IMP-type carbapenemase gene, blaIMP, the third primer set including six oligonucleotides having base sequences of SEQ ID NOs: 11 to 16, respectively,
      (4) a fourth primer set specific to a KPC-type carbapenemase gene, blaKPC, the fourth primer set including six oligonucleotides having base sequences of SEQ ID NOs: 17 to 22, respectively, and
      (5) a fifth primer set specific to a VIM-type carbapenemase gene, blaVIM, the fifth primer set including five oligonucleotides having base sequence of SEQ ID NOs: 23 to 27, respectively,
   wherein the primer set is a LAMP primer set for specifically detecting a target carbapenemase gene by loop-mediated isothermal amplification (LAMP).
<2> A primer set for detecting carbapenemase-producing Enterobacteriaceae, the primer set including:
   at least one selected from the group consisting of
   (6) a sixth primer set specific to an NDM-type carbapenemase gene, blaNDM, the sixth primer set including
      an oligonucleotide having a base sequence of SEQ ID NO: 1,
      an oligonucleotide having a base sequence of SEQ ID NO: 2,
      an oligonucleotide having a base sequence of SEQ ID NO: 3 and being labeled with biotin, and
      an oligonucleotide having a base sequence of SEQ ID NO: 4 and a tag base sequence,
   (7) a seventh primer set specific to an OXA-type carbapenemase gene, blaOXA, the seventh primer set including
      an oligonucleotide having a base sequence of SEQ ID NO: 5,
      an oligonucleotide having a base sequence of SEQ ID NO: 6,
      an oligonucleotide having a base sequence of SEQ ID NO: 7 and
      a tag sequence,
      an oligonucleotide having a base sequence of SEQ ID NO: 8,
      an oligonucleotide having a base sequence of SEQ ID NO: 9, and
      an oligonucleotide having a base sequence of SEQ ID NO: 10 and being labeled with biotin,
   (8) an eighth primer set specific to an IMP-type carbapenemase gene, blaIMP, the eighth primer set including
      an oligonucleotide having a base sequence of SEQ ID NO: 11,
      an oligonucleotide having a base sequence of SEQ ID NO: 12,
      an oligonucleotide having a base sequence of SEQ ID NO: 13 and a tag base sequence,
      an oligonucleotide having a base sequence of SEQ ID NO: 14, and being labeled with biotin,
      an oligonucleotide having a base sequence of SEQ ID NO: 15, and
      an oligonucleotide having a base sequence of SEQ ID NO: 16,
   (9) a ninth primer set specific to a KPC-type carbapenemase gene, blaKPC, the ninth primer set including
      an oligonucleotide having a base sequence of SEQ ID NO: 17,
      an oligonucleotide having a base sequence of SEQ ID NO: 18,
      an oligonucleotide having a base sequence of SEQ ID NO: 19 and a tag base sequence,
      an oligonucleotide having a base sequence of SEQ ID NO: 20, and being labeled with biotin,
      an oligonucleotide having a base sequence of SEQ ID NO: 21, and
      an oligonucleotide having a base sequence of SEQ ID NO: 22, and
   (10) a tenth primer set specific to a VIM-type carbapenemase gene, blaVIM, the tenth primer set including
      an oligonucleotide having a base sequence of SEQ ID NO: 28,
      an oligonucleotide having a base sequence of SEQ ID NO: 29,
      an oligonucleotide having a base sequence of SEQ ID NO: 30 and a tag base sequence,
      an oligonucleotide having a base sequence of SEQ ID NO: 31, and being labeled with biotin,
      an oligonucleotide having a base sequence of SEQ ID NO: 32, and
      an oligonucleotide having a base sequence of SEQ ID NO: 33,
         wherein the primer set is a LAMP DNA chromatography primer set that specifically detects a target carbapenemase gene by loop-mediated isothermal amplification (LAMP) and DNA chromatography.
<3> A detection method for specifically detecting carbapenemase-producing Enterobacteriaceae, the detection method including:
   a step of amplifying a target DNA using a sample that is suspected of containing the carbapenemase-producing Enterobacteriaceae, and the primer set of claim 1 or 2 by LAMP; and
   a step of detecting the amplified DNA.
<4> A detection kit for specifically detecting carbapenemase-producing Enterobacteriaceae, the detection kit including:
   the primer set of claim 1 or 2; and
   an amplifier that amplifies a target DNA by LAMP.

### EFFECTS OF THE INVENTION

According to the present invention, the above various problems existing in the related art can be solved; the above object can be achieved; and a primer set for detecting carbapenemase-producing Enterobacteriaceae, a detection method, and a detection kit, which can specifically detect carbapenemase-producing Enterobacteriaceae even in a system in which a plurality of microbial species are present can be provided.

### BRIEF DESCRIPTION OF THE DRAWINGS

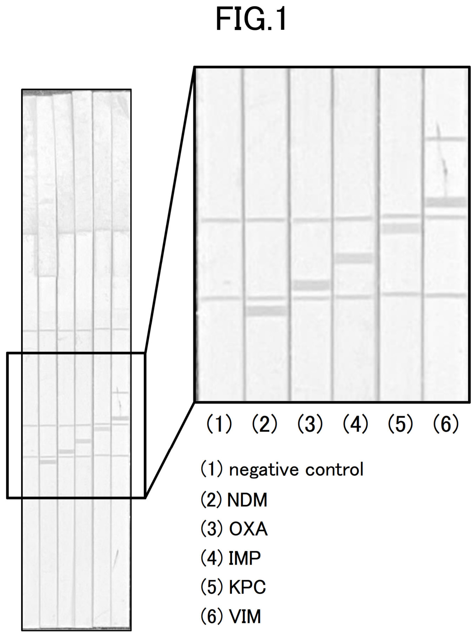

[Fig. 1] Fig. 1 is a view illustrating a detection result of a LAMP DNA chromatography primer set targeting 5 types of carbapenemase genes, NDM-type, OXA-type, IMP-type, KPC-type, and VIM-type, of carbapenemase-producing Enterobacteriaceae.

### DETAILED DESCRIPTION OF THE INVENTION

### (Primer set for detecting carbapenemase-producing Enterobacteriaceae)

The primer set for detecting carbapenemase-producing Enterobacteriaceae of the present invention is a primer set for specifically detecting carbapenemase-producing Enterobacteriaceae. The primer set for detecting carbapenemase-producing Enterobacteriaceae of the present invention is a LAMP primer set including at least one or more of (1) to (5); or a LAMP DNA chromatography primer set including one or more of (6) to (10), which will be described in detail hereinafter.

More specifically, the primer set for detecting carbapenemase-producing Enterobacteriaceae of the present invention is a primer set for specifically detecting each carbapenemase gene (also referred to as a β-lactamase gene) of any of bacterial types, NDM-type, OXA-type, IMP-type, KPC-type, and VIM type, of carbapenemase-producing Enterobacteriaceae.

The above primer sets may be used alone or in combination.

The present inventors have conducted intensive studies to solve the above object, and as a result, the present inventors have found that carbapenemase-producing Enterobacteriaceae can be promptly and easily detected with favorable specificity and high sensitivity even in a system in which a plurality of microbial species are present by preparing an alignment with each type of gene sequences, i.e., an alignment with 19 NDM gene sequences, an alignment with 19 OXA-48 group gene sequences, an alignment with 60 IMP gene sequences, an alignment with 29 KPC gene sequences, and an alignment with 50 VIM gene sequences, preparing primer sets using common sequences as targets, and confirming the sensitivity and specificity of each primer set. Based on this insight, the present invention has been completed.

The primer set, detection kit, and detection method of the present invention can promptly and easily detect carbapenemase-producing Enterobacteriaceae with favorable specificity and high sensitivity even in a system in which a plurality of microbial species are present.

-Carbapenemase-producing Enterobacteriaceae-Carbapenemase-producing Enterobacteriaceae are bacteria of the family Enterobacteriaceae, which produce carbapenemase that decomposes most of β-lactam antibiotics including carbapenems, and the cell types of CPE can be classified by the Ambler classification (genotypes, etc.) according to the carbapenemase genes and amino acid sequences based on the substrate specificity, or by enzyme types.

Examples of main cell types of the carbapenemase-producing Enterobacteriaceae detected in Japan include: the NDM type; the OXA type, such as OXA-48 group, OXA-181 group, and the like; the IMP type, such as IMP-7 group, IMP-1 group, IMP-6 group, IMP-11 group, IMP-34 group, IMP-52 group, and the like; the KPC type; the VIM type, such as VIM-2 group and the like; etc.

### <LAMP primer set>

The LAMP primer set is a primer set for detecting carbapenemase-producing Enterobacteriaceae, is a LAMP primer set that specifically detects a target carbapenemase gene by loop-mediated isothermal amplification (LAMP), and includes at least one selected from the group consisting of the following (1) to (5) first to fifth primer sets as presented in Table 1 below.

As the LAMP primer set, one of the first to fifth primer sets may be used alone, or two or more of the first to fifth primer sets may be used in combination.

**[Table 1]**

| LAMP primer set | | | |
|---|---|---|---|
| Gene | Primer | Base sequence | SEQ ID NO |
| *bla*NDM | F3 | TCGATACCGCCTGGACC | 1 |
| | B3 | TCGACAACGCATTGGCATA | 2 |
| | FIP | GCGCGACCGGCAGGTTGATGATGACCAGACCGCCCAG | 3 |
| | BIP | CGGTGGTGACTCACGCGCAAGTCGCAATCCCCGCC | 4 |
| *bla*OXA | F3 | AATAGCTTGATCGCCCTC | 5 |
| | B3 | CCATAATCGAAAGCATGTAGC | 6 |
| | FIP | GATTCCAAGTGGCGATATCGCGGCGTGGTTAAGGATGA AC | 7 |
| | BIP | TAATCACCGCGATGAAATATTCAGTCTTGCTCATACGTG CCTC | 8 |
| | LF | GCGTCTGTCCATCCCACTTA | 9 |
| | LB | GAATTTGCCCGCCAAATTGG | 10 |
| *bla*IMP | F3 | GCGTTGTTCCTAAACATGG | 11 |
| | B3 | TTGTTAATTCAGATGCATACGT | 12 |
| | FIP | TCCACAAACCAAGTGACTAACTTTTTGGTTCTTGTAAAT GCTGAGG | 13 |
| | BIP | CGTGGCTATAAAATAAAAGGCAGCAGATCGAGAATTAAG CCACTC | 14 |
| | LF | AGTATCTTTAGCCGTAAATGGAGTG | 15 |
| | LB | CGACAGCACGGGCGGAATA | 16 |
| *bla*KPC | F3 | ACTCCGCCATCCCAAGC | 17 |
| | B3 | TCCGACTGCCCAGTCTG | 18 |
| | FIP | TGCAGAGCCCAGTGTCAGTTTTGATGCGCGCGATACCTC | 19 |
| | BIP | GCAGCGGCAGCAGTTTGTTGCGGATGCGGTGGTTGC | 20 |
| | LF | GTAAGCTTTCCGTCACGGCG | 21 |
| | LB | TTGGCTAAAGGGAAACACGACC | 22 |
| *bla*VIM | F3 | AATTCCGGTCGGAGAGGT | 23 |
| | B3 | AAAGTGCGTGGAGACTGC | 24 |
| | FIP | CATTGGACGGGTAGACCGCGCCAGATTGCCGATGGTGT | 25 |
| | BIP | TGATTGATACAGCGTGGGGTGCACGGGAAGTCCAATTT GCT | 26 |
| | LF | CCATCAAACGACTGCGTTGC | 27 |

(1) The first primer set is specific to an NDM-type carbapenemase gene, blaNDM, and includes four oligonucleotides having base sequences of SEQ ID NOs: 1 to 4, respectively, specifically including an F3 primer (SEQ ID NO: 1) and a B3 primer (SEQ ID NO: 2) as outer primers specific to blaNDM, and an FIP primer (SEQ ID NO: 3) and a BIP primer (SEQ ID NO: 4) as inner primers.
(2) The second primer set is specific to an OXA-type carbapenemase gene, blaOXA, and includes six oligonucleotides having base sequences of SEQ ID NOs: 5 to 10, respectively, specifically including an F3 primer (SEQ ID NO: 5) and a B3 primer (SEQ ID NO: 6) as outer primers specific to blaOXA, an FIP primer (SEQ ID NO: 7) and a BIP primer (SEQ ID NO: 8) as inner primers, and an LF primer (SEQ ID NO: 9) and an LB primer (SEQ ID NO: 10) as loop primers.
(3) The third primer set is specific to an IMP-type carbapenemase gene, blaIMP, and includes six oligonucleotides having base sequences of SEQ ID NOs: 11 to 16, respectively, specifically including an F3 primer (SEQ ID NO: 11) and a B3 primer (SEQ ID NO: 12) as outer primers specific to blaIMP, an FIP primer (SEQ ID NO: 13) and a BIP primer (SEQ ID NO: 14) as inner primers, and an LF primer (SEQ ID NO: 15) and an LB primer (SEQ ID NO: 16) as loop primers.
(4) The fourth primer set is specific to a KPC-type carbapenemase gene, blaKPC, and includes six oligonucleotides having base sequences of SEQ ID NOs: 17 to 22, respectively, specifically including an F3 primer (SEQ ID NO: 17) and a B3 primer (SEQ ID NO: 18) as outer primers specific to blaKPC, an FIP primer (SEQ ID NO: 19) and a BIP primer (SEQ ID NO: 20) as inner primers, and an LF primer (SEQ ID NO: 21) and an LB primer (SEQ ID NO: 22) as loop primers.
(5) The fifth primer set is specific to a VIM-type carbapenemase gene, blaVIM, and includes five oligonucleotides having base sequences of SEQ ID NOs: 23 to 27, respectively, specifically including an F3 primer (SEQ ID NO: 23) and a B3 primer (SEQ ID NO: 24) as outer primers specific to blaVIM, an FIP primer (SEQ ID NO: 25) and a BIP primer (SEQ ID NO: 26) as inner primers, and an LF primer (SEQ ID NO: 27) as a loop primer.

As described above, a gene amplification detection method, such as PCR has been desired as a method for promptly and assuredly detecting CPE. However, facilities capable of performing CPE detection by PCR are limited. Accordingly, the present inventors have selected and created a LAMP primer set that specifically detects a target carbapenemase gene by loop-mediated isothermal amplification (LAMP) that enables isothermal gene amplification.

LAMP is a method in which amplification is performed by a strand displacement reaction using four to six primers obtained by selecting and combining six regions from the sequence of the target gene (see, for example, Nucleic Acids Research, Vol. 28, 12, 2000, etc.). LAMP does not require a temperature cycle like PCR, and can specifically amplify a gene by an isothermal reaction at 60°C to 65°C for 30 minutes to 60 minutes. Whether DNA amplification is performed by the LAMP reaction can be determined by visually observing or measuring the turbidity due to magnesium pyrophosphate, which is generated as a by-product of the DNA amplification, by a turbidimeter.

The LAMP primer set of the present embodiment can promptly and easily detect carbapenemase-producing Enterobacteriaceae with favorable specificity and high sensitivity even in a system in which a plurality of microbial species are present.

### <LAMP DNA chromatography primer set>

The LAMP DNA chromatography primer set is a primer set for detecting carbapenemase-producing Enterobacteriaceae, is a LAMP DNA chromatography primer set that specifically detects a target carbapenemase gene by loop-mediated isothermal amplification (LAMP) and DNA chromatography, and includes at least one selected from the group consisting of the following (6) to (10) sixth to tenth primer sets.

As the LAMP DNA chromatography primer set, one of the sixth to tenth primer sets may be used alone, or two or more of the sixth to tenth primer sets may be used in combination.

**[Table 2]**

| LAMP DNA chromatography primer set | | | |
|---|---|---|---|
| Gene | Primer | Base sequence | SEQ ID NO |
| *bla*NDM | F3 | TCGATACCGCCTGGACC | 1 |
| | B3 | TCGACAACGCATTGGCATA | 2 |
| | FIP-biotin | GCGCGACCGGCAGGTTGATGATGACCAGACCGCCCAG | 3 |
| | BIP-tag | CGGTGGTGACTCACGCGCAAGTCGCAATCCCCGCC | 4 |
| *bla*OXA | F3 | AATAGCTTGATCGCCCTC | 5 |
| | B3 | CCATAATCGAAAGCATGTAGC | 6 |
| | FIP-tag | GATTCCAAGTGGCGATATCGCGGCGTGGTTAAGGATGA AC | 7 |
| | BIP | TAATCACCGCGATGAAATATTCAGTCTTGCTCATACGTG CCTC | 8 |
| | LF | GCGTCTGTCCATCCCACTTA | 9 |
| | LB-biotin | GAATTTGCCCGCCAAATTGG | 10 |
| *bla*IMP | F3 | GCGTTGTTCCTAAACATGG | 11 |
| | B3 | TTGTTAATTCAGATGCATACGT | 12 |
| | FIP-tag | TCCACAAACCAAGTGACTAACTTTTTGGTTCTTGTAAAT GCTGAGG | 13 |
| | BIP-biotin | CGTGGCTATAAAATAAAAGGCAGCAGATCGAGAATTAAG CCACTC | 14 |
| | LF | AGTATCTTTAGCCGTAAATGGAGTG | 15 |
| | LB | CGACAGCACGGGCGGAATA | 16 |
| *bla*KPC | F3 | ACTCCGCCATCCCAAGC | 17 |
| | B3 | TCCGACTGCCCAGTCTG | 18 |
| | FIP-tag | TGCAGAGCCCAGTGTCAGTTTTGATGCGCGCGATACCTC | 19 |
| | BIP-biotin | GCAGCGGCAGCAGTTTGTTGCGGATGCGGTGGTTGC | 20 |
| | LF | GTAAGCTTTCCGTCACGGCG | 21 |
| | LB | TTGGCTAAAGGGAAACACGACC | 22 |
| *bla*VIM | F3 | TGGTCGCATATCGCAACG | 28 |
| | B3 | GCCCGAAGGACATCAACG | 29 |
| | FIP-tag | GCACCCCACGCTGTATCAATCAGGTCTACCCGTCCAATG GT | 30 |
| | BIP-biotin | AACACAGCGGCACTTCTCGCTGAAAGTGCGTGGAGACTG | 31 |
| | LF | GCAACTCATCACCATCACGGACAA | 32 |
| | LB | GCAAATTGGACTTCCCGTAACG | 33 |

(6) The sixth primer set is specific to an NDM-type carbapenemase gene, blaNDM, and includes
an oligonucleotide having a base sequence of SEQ ID NO: 1,
an oligonucleotide having a base sequence of SEQ ID NO: 2,
an oligonucleotide having a base sequence of SEQ ID NO: 3 and being labeled with biotin, and an oligonucleotide having a base sequence of SEQ ID NO: 4 and a tag base sequence.

Specifically, the sixth primer set includes an F3 primer (SEQ ID NO: 1) and a B3 primer (SEQ ID NO: 2) as outer primers specific to blaNDM, and a biotin-labeled FIP primer (SEQ ID NO: 3) and a BIP primer (SEQ ID NO: 4) having a tag sequence (base sequence) as inner primers.

(7) The seventh primer set is specific to an OXA-type carbapenemase gene, blaOXA, and includes
an oligonucleotide having a base sequence of SEQ ID NO: 5,
an oligonucleotide having a base sequence of SEQ ID NO: 6,
an oligonucleotide having a base sequence of SEQ ID NO: 7 and
a tag base sequence,
an oligonucleotide having a base sequence of SEQ ID NO: 8,
an oligonucleotide having a base sequence of SEQ ID NO: 9, and
an oligonucleotide having a base sequence of SEQ ID NO: 10 and being labeled with biotin.

Specifically, the seventh primer set includes an F3 primer (SEQ ID NO: 5) and a B3 primer (SEQ ID NO: 6) as outer primers specific to blaOXA, an FIP primer (SEQ ID NO: 7) having a tag sequence (base sequence) and a BIP primer (SEQ ID NO: 8) as inner primers, and an LF primer (SEQ ID NO: 9) and a biotin-labeled LB primer (SEQ ID NO: 10) as loop primers.

(8) The eighth primer set is specific to an IMP-type carbapenemase gene, blaIMP, and includes
an oligonucleotide having a base sequence of SEQ ID NO: 11,
an oligonucleotide having a base sequence of SEQ ID NO: 12,
an oligonucleotide having a base sequence of SEQ ID NO: 13
and a tag base sequence,
an oligonucleotide having a base sequence of SEQ ID NO: 14, and being labeled with biotin,
an oligonucleotide having a base sequence of SEQ ID NO: 15,
   and
an oligonucleotide having a base sequence of SEQ ID NO: 16.

Specifically, the eighth primer set includes an F3 primer (SEQ ID NO: 11) and a B3 primer (SEQ ID NO: 12) as outer primers specific to blaIMP, an FIP primer (SEQ ID NO: 13) having a tag sequence (base sequence) and a biotin-labeled BIP primer (SEQ ID NO: 14) as inner primers, and an LF primer (SEQ ID NO: 15) and an LB primer (SEQ ID NO: 16) as loop primers.

(9) The ninth primer set is specific to a KPC-type carbapenemase gene, blaKPC, and includes
an oligonucleotide having a base sequence of SEQ ID NO: 17,
an oligonucleotide having a base sequence of SEQ ID NO: 18,
an oligonucleotide having a base sequence of SEQ ID NO: 19 and a tag base sequence,
an oligonucleotide having a base sequence of SEQ ID NO: 20 and being labeled with biotin,
an oligonucleotide having a base sequence of SEQ ID NO: 21, and
an oligonucleotide having a base sequence of SEQ ID NO: 22.

Specifically, the nineth primer set includes an F3 primer (SEQ ID NO: 17) and a B3 primer (SEQ ID NO: 18) as outer primers specific to blaKPC, an FIP primer (SEQ ID NO: 19) having a tag sequence (base sequence) and a biotin-labeled BIP primer (SEQ ID NO: 20) as inner primers, and an LF primer (SEQ ID NO: 21) and an LB primer (SEQ ID NO: 22) as loop primers.

(10) The tenth primer set is specific to a VIM-type carbapenemase gene, blaVIM, and includes
an oligonucleotide having a base sequence of SEQ ID NO: 28,
an oligonucleotide having a base sequence of SEQ ID NO: 29,
an oligonucleotide having a base sequence of SEQ ID NO: 30 and a tag base sequence,
an oligonucleotide having a base sequence of SEQ ID NO: 31 and being labeled with biotin,
an oligonucleotide having a base sequence of SEQ ID NO: 32, and
an oligonucleotide having a base sequence of SEQ ID NO: 33.

Specifically, the tenth primer set includes an F3 primer (SEQ ID NO: 28) and a B3 primer (SEQ ID NO: 29) as outer primers specific to blaVIM, an FIP primer (SEQ ID NO: 30) having a tag sequence (base sequence) and a biotin-labeled BIP primer (SEQ ID NO: 31) as inner primers, and an LF primer (SEQ ID NO: 32) and an LB primer (SEQ ID NO: 33) as loop primers.

As described above, LAMP can be performed by visually observing or measuring the turbidity due to magnesium pyrophosphate, which is generated as a by-product of DNA amplification, using a turbidimeter. However, the determination by visual observation causes a problem that a different determination result may be provided to the same result depending on an inspector. In addition, an expensive device is required for the determination using the turbidimeter, and it is expected that there is a case where use of a turbidimeter is not possible when detection is performed in various regions.

Even when there is a restriction in availability of a detection device, the LAMP DNA chromatography primer set of the present embodiment can promptly and easily detect carbapenemase-producing Enterobacteriaceae with favorable specificity and high sensitivity by using DNA chromatography even in a system in which a plurality of microbial species are present.

### (Detection method)

The detection method of the present invention is a detection method for specifically detecting carbapenemase-producing Enterobacteriaceae. The detection method includes a step of amplifying a target DNA using a sample that is suspected of containing the carbapenemase-producing Enterobacteriaceae and the above-described primer set of the present invention by LAMP and a step of detecting the amplified DNA, and may further include other steps, as necessary.

### <Amplification step>

The amplification step is a step of amplifying a target DNA using a sample that is suspected of containing the carbapenemase-producing Enterobacteriaceae and the above-described primer set of the present invention by LAMP.

The sample is not particularly limited, and may be appropriately selected according to the intended purpose. Examples of the sample include a sample including Enterobacteriaceae collected from a patient who is suspected of having an infection, a sample including Enterobacteriaceae collected from a patient undergoing a treatment process, and a sample collected from any site suspected of being contaminated with CPE.

The method of amplifying the target DNA by LAMP is not particularly limited, and reaction conditions are appropriately selected according to the intended purpose in accordance with known LAMP procedures in the related art. Examples of the method include a method in which gene-specific amplification is performed by an isothermal reaction at 60°C to 65°C for 30 minutes to 60 minutes, followed by inactivating the enzyme, for example, at 80°C or higher, and the like.

The method of preparing a template DNA for the amplification of the target DNA by LAMP is not particularly limited, and may be appropriately selected from methods known in the related art according to the intended purpose. Examples of the method include a method of extracting a genomic DNA of a sample.

### <Detection step>

The detection step is a step of detecting the amplified DNA.

The method of detecting the amplified DNA is not particularly limited, and may be appropriately selected from methods known in the related art according to the intended purpose.

In the case where the primer set is a LAMP primer set, examples of the method of detecting the amplified DNA include: a method in which a positive result or negative result of DNA amplification is determined by measuring turbidity of a reaction product from the DNA amplification using Loopamp EXIA (EIKEN CHEMICAL CO., LTD.); a method of visually confirming turbidity of a reaction product; and the like.

In the case where the primer set is a LAMP DNA chromatography primer set, the amplified DNA can be detected according to a known method of DNA chromatography. For example, the amplified DNA can be detected, for example, by a dipstick DNA chromatography strip (C-PAS) designed by STH-PAS System (TBA (Tohoku Bio Array) Co., Ltd.).

Fig. 1 illustrates a detection example by LAMP DNA chromatography. Fig. 1 illustrates the detection results obtained with the LAMP DNA chromatography primer set targeting five types of carbapenemase genes, NDM-type, OXA-type, IMP-type, KPC-type, and VIM-type, of carbapenemase-producing Enterobacteriaceae.

The LAMP DNA chromatography primer set that detects each cell type includes a tag sequence specific to a corresponding cell type. Thus, an oligonucleotide having a base sequence complementary to the specific tag sequence is immobilized on a corresponding detection position, which varies depending on a cell type to be detected, in DNA chromatography for detecting each of the cell types. In addition, the complementary oligonucleotide immobilized on the DNA chromatography and the amplified product obtained by the LAMP DNA chromatography primer set are bound, and the biotin labeling in the amplified product is detected by avidin color beads. As illustrated in Fig. 1, cell types can be distinguishably detected by confirming a blue positive band at a position corresponding to each gene group on the DNA chromatography (C-PAS).

By using the detection kit of the present embodiment, the detection method can be suitably performed.

### (Detection kit)

The detection kit of the present invention is a detection kit for specifically detecting carbapenemase-producing Enterobacteriaceae, and includes the above-described primer set of the present invention and an amplifier that amplifies a target DNA by LAMP, preferably includes a detector that specifically detects gene amplification performed with the primer set, and may further include other devices, as necessary.

### <Amplifier>

The amplifier is a device that amplifies a target DNA by LAMP. The amplifier is not particularly limited, and may be appropriately selected from known molecules according to the intended purpose. Examples of the amplifier include: reagents, such as strand displacement DNA polymerase, DNA substrates, buffer solutions, and the like; a heat retaining device capable of retaining heat at a constant temperature; and the like.

### <Detector>

The detector is a device that specifically detects the gene amplification performed with the primer set. The detector is not particularly limited, and may be appropriately selected from known molecules according to the intended purpose.

The device for detecting the amplified DNA is not particularly limited, and may be appropriately selected from known devices in the art according to the intended purpose.

In the case where the primer set is a LAMP primer set, examples of the detector include a device of determining a positive or negative result of DNA amplification by measuring turbidity of a reaction product from the DNA amplification using Loopamp EXIA (EIKEN CHEMICAL CO., LTD.), a device of visually confirming turbidity of a reaction product, and the like.

In the case where the primer set is a LAMP DNA chromatography primer set, known DNA chromatography may be appropriately selected. Examples of the detector include a dipstick DNA chromatography strip (C-PAS) designed by STH-PAS System (TBA Co., Ltd.), and the like.

By using the detection kit of the present embodiment, the detection step and the detection method can be suitably performed.

### Examples

The present invention will be more concretely described through Examples hereinafter, but the present invention is not limited by Examples below.

### <Production of LAMP primer set>

There are five major types (NDM-type, OXA-type, IMP-type, KPC-type, and VIM-type) of carbapenemase genes that are carbapenem resistant genes. The alignment was prepared with each type of gene sequences, i.e., the alignment with 19 NDM sequences, the alignment with 19 OXA-48 group sequences, the alignment with 60 IMP sequences, the alignment with 29 KPC sequences, and the alignment with 50 VIM sequences, and a plurality of LAMP primer sets were produced with common sequences as targets using Primer Explorer V5 software (EIKEN CHEMICAL CO., LTD.).

### <Preparation of genomic DNA of bacterial isolate>

A genomic DNA of each bacterial isolate was extracted using QIAamp DNA mini kit (Qiagen, Germany) according to the manufacturer's instructions. The LAMP reaction was carried out using Loopamp (registered trademark) DNA amplification kit D (EIKEN CHEMICAL CO., LTD.) according to the manufacturer's instructions.

### <Detection of bacterial isolate by LAMP>

The LAMP reaction was carried out in a total volume of 25 µL including 1.6 µM of each inner primer (FIP primer and BIP primer), 0.2 µM of each outer primer (F3 primer and B3 primer), 0.8 µM of each loop primer (LF primer and LB primer), and 1 µL of a target DNA template.

In addition, the LAMP reaction was performed also for each of the NDM-type and VIM-type in a total volume of 25 µL by adjusting the total volume with water.

The reaction was performed by amplifying the DNA at 65°C for 40 minutes using a real-time turbidity measuring device Loopamp EXIA (EIKEN CHEMICAL CO., LTD.), followed by inactivating the enzyme at 80°C for 5 minutes. A positive result of the LAMP reaction was automatically judged based on the turbidity by Loopamp EXIA (EIKEN CHEMICAL CO., LTD.). The sensitivity and specificity evaluation test using the DNA of bacterial isolate was repeated three times to ensure reproducibility.

### <Detection of bacterial isolate by LAMP DNA chromatography>

A multiplex detection method for identifying five types of carbapenemase gene groups was developed using STH-PAS System, which was a gene testing method of TBA Co., Ltd.

Commercial products (TBA Co., Ltd., Japan) of a dipstick DNA chromatography strip (C-PAS) and reagents were acquired. In LAMP DNA chromatography, the FIP primer or the BIP primer was labeled with a tag sequence (gene sequence), and the FIP primer, the BIP primer, or the LoopB primer was labeled with biotin. The reactions of LAMP DNA chromatography reaction were performed on a heat block. The amplification was performed by LAMP at 65°C for 40 minutes, thereby labeling the LAMP product with the tagged sequence and biotin. The C-PAS F8 membrane strip (TBA Co., Ltd.) was inserted into 21 µL of the reaction mixture including 10 µL of a developing solution (TBA Co., Ltd.), 9 µL of distilled water, 1 µL of the LAMP product, and 1 µL of avidin-coated blue beads (TBA Co., Ltd.).

Fig. 1 illustrates the detection result with the LAMP DNA chromatography primer set targeting five carbapenemase genes, which are NDM-type, OXA-type, IMP-type, KPC-type, and VIM-type, of carbapenemase-producing Enterobacteriaceae.

As demonstrated in Fig. 1, the LAMP product labeled with the avidin-coated blue beads was captured by the complementary gene sequence printed on the strip membrane via the tag sequence attached to the end of the LAMP product, and appeared as a blue line on the C-PAS. On detection lines of the C-PAS, base sequences (named c-tags 1-5) complementary to five tags of different base sequences, respectively, were immobilized in advance. The blue positive band was observed at the position of the C-PAS corresponding to each of the gene groups with all the samples including the carbapenemase gene. The sensitivity evaluation test and the specificity evaluation test using the DNA of bacterial isolate were repeated three times to ensure reproducibility.

### <Examination of sensitivity and specificity>

Using the DNA extracted from bacteria in which the presence of 73 carbapenem resistant genes (plasmids) (16 NDM genes, 19 OXA genes, 20 IMP genes, 14 KPC genes, and 9 VIM genes) was known, whether or not a positive reaction occurred (sensitivity) with the target plasmid, and no reaction occurred with other plasmids or bacterial genes (specificity) was determined by LAMP and by LAMP DNA chromatography. The results are presented in Tables 3 and 4.

It was confirmed from the results of Table 3 that 73 types of carbapenemase-producing Enterobacteriaceae could be detected with the LAMP primer set at the sensitivity of 93% or greater and the specificity of 98% or greater.

In addition, it was confirmed from the results of Table 4 that 73 types of carbapenemase-producing Enterobacteriaceae could be detected with the LAMP DNA chromatography primer set at the sensitivity of 100% or greater and the specificity of 97% or greater.

**[Table 3]**

| LAMP | | |
|---|---|---|
| Genotype | Sensitivity | Specificity |
| NDM | 100% | 100% |
| OXA | 100% | 100% |
| IMP | 95% | 100% |
| KPC | 93% | 98% |
| VIM | 100% | 100% |

**[Table 4]**

| LAMP DNA chromatography | | |
|---|---|---|
| Genotype | Sensitivity | Specificity |
| NDM | 100% | 100% |
| OXA | 100% | 98% |
| IMP | 100% | 100% |
| KPC | 100% | 97% |
| VIM | 100% | 100% |

The present international application claims priority based on Japanese Patent Application No. 2023-091020 filed on June 1, 2023, and the entire contents of Japanese Patent Application No. 2023-091020 are incorporated herein by reference.

[Sequence Listing] KU24001PCT

## Claims

1. A primer set for detecting carbapenemase-producing Enterobacteriaceae, the primer set comprising:
at least one selected from the group consisting of
(1) a first primer set specific to an NDM-type carbapenemase gene, blaNDM, the first primer set including four oligonucleotides having having base sequences of SEQ ID NOs: 1 to 4, respectively,
(2) a second primer set specific to an OXA-type carbapenemase gene, blaOXA, the second primer set including six oligonucleotides having base sequences of SEQ ID NOs: 5 to 10, respectively,
(3) a third primer set specific to an IMP-type carbapenemase gene, blaIMP, the third primer set including six oligonucleotides having base sequences of SEQ ID NOs: 11 to 16, respectively,
(4) a fourth primer set specific to a KPC-type carbapenemase gene, blaKPC, the fourth primer set including six oligonucleotides having base sequences of SEQ ID NOs: 17 to 22, respectively, and
(5) a fifth primer set specific to a VIM-type carbapenemase gene, blaVIM, the fifth primer set including five oligonucleotides having base sequence of SEQ ID NOs: 23 to 27, respectively,
wherein the primer set is a LAMP primer set for specifically detecting a target carbapenemase gene by loop-mediated isothermal amplification (LAMP).

2. A primer set for detecting carbapenemase-producing Enterobacteriaceae, the primer set comprising:
at least one selected from the group consisting of
(6) a sixth primer set specific to an NDM-type carbapenemase gene, blaNDM, the sixth primer set including
an oligonucleotide having a base sequence of SEQ ID NO: 1,
an oligonucleotide having a base sequence of SEQ ID NO: 2,
an oligonucleotide having a base sequence of SEQ ID NO: 3 and being labeled with biotin, and
an oligonucleotide having a base sequence of SEQ ID NO: 4 and a tag base sequence,
(7) a seventh primer set specific to an OXA-type carbapenemase gene, blaOXA, the seventh primer set including
an oligonucleotide having a base sequence of SEQ ID NO: 5,
an oligonucleotide having a base sequence of SEQ ID NO: 6,
an oligonucleotide having a base sequence of SEQ ID NO: 7 and a tag base sequence,
an oligonucleotide having a base sequence of SEQ ID NO: 8,
an oligonucleotide having a base sequence of SEQ ID NO: 9, and
an oligonucleotide having a base sequence of SEQ ID NO: 10 and being labeled with biotin,
(8) an eighth primer set specific to an IMP-type carbapenemase gene, blaIMP, the eighth primer set including
an oligonucleotide having a base sequence of SEQ ID NO: 11,
an oligonucleotide having a base sequence of SEQ ID NO: 12,
an oligonucleotide having a base sequence of SEQ ID NO: 13 and a tag base sequence,
an oligonucleotide having a base sequence of SEQ ID NO: 14, and being labeled with biotin,
an oligonucleotide having a base sequence of SEQ ID NO: 15, and
an oligonucleotide having a base sequence of SEQ ID NO: 16,
(9) a ninth primer set specific to a KPC-type carbapenemase gene, blaKPC, the ninth primer set including
an oligonucleotide having a base sequence of SEQ ID NO: 17,
an oligonucleotide having a base sequence of SEQ ID NO: 18,
an oligonucleotide having a base sequence of SEQ ID NO: 19 and a tag base sequence,
an oligonucleotide having a base sequence of SEQ ID NO: 20, and being labeled with biotin,
an oligonucleotide having a base sequence of SEQ ID NO: 21, and
an oligonucleotide having a base sequence of SEQ ID NO: 22, and
(10) a tenth primer set specific to a VIM-type carbapenemase gene, blaVIM, the tenth primer set including
an oligonucleotide having a base sequence of SEQ ID NO: 28,
an oligonucleotide having a base sequence of SEQ ID NO: 29,
an oligonucleotide having a base sequence of SEQ ID NO: 30 and a tag base sequence,
an oligonucleotide having a base sequence of SEQ ID NO: 31, and being labeled with biotin,
an oligonucleotide having a base sequence of SEQ ID NO: 32, and
an oligonucleotide having a base sequence of SEQ ID NO: 33,
wherein the primer set is a LAMP DNA chromatography primer set that specifically detects a target carbapenemase gene by loop-mediated isothermal amplification (LAMP) and DNA chromatography.

3. A detection method for specifically detecting carbapenemase-producing Enterobacteriaceae, the detection method comprising:
a step of amplifying a target DNA using a sample that is suspected of containing the carbapenemase-producing Enterobacteriaceae, and the primer set of claim 1 or 2, by LAMP; and
a step of detecting the amplified DNA.

4. A detection kit for specifically detecting carbapenemase-producing Enterobacteriaceae, the detection kit comprising:
the primer set of claim 1 or 2; and
an amplifier that amplifies a target DNA by LAMP.
